# EUROPÄISCHE PATENTSCHRIFT

(11) **EP 2 510 866 B1**
(45) Veröffentlichungstag und Bekanntmachung des Hinweises auf die Patenterteilung: **27.07.2016**
(21) Anmeldenummer: 12163218.6
(22) Anmeldetag: 04.04.2012
(51) Int. Cl.: A61B 1/00, G02B 23/24, A61B 1/04

(54) **Endoskop**
Endoscope
Endoscope

(30) Priorität: 12.04.2011 DE 102011007190
(43) Veröffentlichungstag der Anmeldung: 17.10.2012
(73) Patentinhaber: Henke-Sass, Wolf GmbH, 78532 Tuttlingen (DE)
(72) Erfinder: Rehe, Oliver, 78532 Tuttlingen (DE)
(74) Vertreter: Patentanwälte Geyer, Fehners & Partner mbB

(56) Entgegenhaltungen:
- DE-A1-102009 025 660
- DE-B3-102009 049 143
- US-A- 3 856 000
- US-A- 4 140 364
- US-A1- 2003 092 966
- US-A1- 2004 236 183
- US-A1- 2009 306 474

## Beschreibung

Die vorliegende Erfindung betrifft ein Endoskop mit einem ersten Rohr, einem am distalen Ende des ersten Rohrs angeordneten Deckglas sowie einer im ersten Rohr angeordneten Abbildungsoptik, die ein vor dem Deckglas befindliches Objekt als Bild abbildet.

Das Deckglas ist häufig als planparallele Platte ausgebildet. Da zur Abbildung des Objektes mittels der Abbildungsoptik Lichtstrahlen vom Objekt durch das Deckglas laufen, besteht die Schwierigkeit, daß diese Lichtstrahlen in Abhängigkeit des an der Außenseite des Deckglases vorliegenden Mediums (z.B. Luft, Flüssigkeit, etc.) unterschiedlich stark gebrochen werden. Dies wirkt sich natürlich auf die Abbildungsqualität aus.

Wenn das Endoskop als Endoskop mit variabler Blickrichtung ausgebildet ist, liegt zusätzlich eine Abhängigkeit der Brechung der Lichtstrahlen von der Blickrichtung vor.

Dokument DE 10 2009 025 660 A1, das den nächstliegenden Stand der Technik bildet offenbart in diesem Zusammenhang die Präambel von Anspruch 1.

Ausgehend hiervon ist es Aufgabe der Erfindung, ein Endoskop der eingangs genannten Art so auszubilden, daß eine bessere Abbildung möglich ist.

Die Aufgabe wird bei einem Endoskop der eingangs genannten Art dadurch gelöst, daß das Deckglas eine sphärisch gekrümmte, konkave Innenseite, eine sphärisch gekrümmte, konvexe Außenseite sowie eine konstante Dicke aufweist.

Dadurch treten in vorteilhafter Weise die von jedem Objektpunkt ausgehenden und für die Bilderzeugung benutzten Lichtstrahlen senkrecht bzw. im wesentlichen senkrecht durch das Deckglas hindurch, so daß das Deckglas keine oder nur eine sehr geringe Brechung bewirkt. Als Folge davon ist der Einfluß des Mediums an der Außenseite des Deckglases bei dem erfindungsgemäßen Endoskop gegenüber einem bekannten Endoskop sehr stark reduziert.

Bei dem erfindungsgemäßen Endoskop kann sich das Deckglas in einer ersten Ebene über einen ersten Winkelbereich erstrecken und sich in einer zweiten Ebene, die senkrecht zur ersten Ebene ist, über einen zweiten Winkelbereich erstrecken, wobei der erste Winkelbereich größer ist als der zweite Winkelbereich. Insbesondere kann der erste Winkelbereich mindestens 1,5 mal oder mindestens 2 mal so groß sein wie der zweite Winkelbereich. Dies ist insbesondere bei einem Endoskop mit variabler Blickrichtung von Vorteil, wobei der erste Winkelbereich bevorzugt so gewählt ist, daß alle möglichen Blickrichtungen abgedeckt sind.

Bei dem erfindungsgemäßen Endoskop kann die Innenseite entspiegelt sein. Ferner kann zusätzlich oder alternativ die Außenseite entspiegelt sein. Dies verbessert die Abbildungseigenschaften.

Ferner kann der Rand des Deckglases mit dem distalen Ende des ersten Rohres verlötet sein. Damit kann das distale Ende des ersten Rohres hermetisch dicht ausgebildet sein. Insbesondere kann das Endoskop dadurch autoklavierbar sein.

Natürlich ist auch jede andere Art der Verbindung des Deckglases mit dem distalen Ende des ersten Rohrs möglich. So kann z.B. das Deckglas mit dem distalen Ende des ersten Rohrs verklebt sein.

Das Deckglas kann aus Kunststoff oder Glas gebildet sein. Es ist bevorzugt aus Saphirglas gebildet.

Bei dem erfindungsgemäßen Endoskop kann die Abbildungsoptik ein schwenkbar gelagertes Umlenkelement aufweisen, mit dem die Blickrichtung durch das Deckglas einstellbar ist. Bei dem Umlenkelement kann es sich insbesondere um ein Umlenkprisma oder einen Spiegel handeln. Die Drehachse des Umlenkelementes liegt bevorzugt senkrecht zur Längsrichtung des ersten Rohrs.

Ferner kann das Umlenkelement in einem drehbar gelagerten Halter befestigt sein und mittels einem Zugrohr, das zusammen mit dem ersten Rohr in einem Endoskopschaft des Endoskops positioniert ist, um seine Drehachse gedreht werden. Dazu kann beispielsweise das proximale Ende des Zugrohrs über ein an einem Handgriff des Endoskops angeordnetes Betätigungselement axial bewegt werden. Insbesondere kann das Betätigungselement über ein Getriebe mit dem Zugrohr mechanisch verbunden sein. Das Getriebe kann bevorzugt so ausgebildet sein, daß es eine Drehbewegung des Betätigungselementes in eine axiale Bewegung des Zugrohrs umsetzt.

Im oder am ersten Rohr ist bevorzugt noch ein Beleuchtungskanal vorgesehen, über den das abzubildende Objekt beleuchtet werden kann. Die Beleuchtung kann beispielsweise mittels Lichtleitfasern erfolgen, die am Handgriff des Endoskops mit Licht beaufschlagt werden können. Natürlich sind auch andere Arten der Beleuchtung möglich. Insbesondere kann eine Lichtquelle (z.B. ein oder mehrere LED-Dioden) am distalen Ende des ersten Endoskopschaftes zur Beleuchtung vorgesehen sein.

Bei dem erfindungsgemäßen Endoskop können die Mittelpunkte der Krümmungsradien der Innen- und Außenseite zusammenfallen und auf oder nahe der Schwenkachse des Umlenkelementes und/oder in oder nahe einer die Strahlumlenkung bewirkenden Fläche des Umlenkelementes liegen. Damit können ausgezeichnete optische Abbildungseigenschaften erreicht werden.

Bei dem erfindungsgemäßen Endoskop kann das Deckglas eine die Innen- und Außenseite verbindende, ebene Randfläche aufweisen, die gegenüber den Krümmungsradien der Innen- und Außenseite geneigt ist.

Insbesondere kann die Randfläche einen linken und rechten Abschnitt aufweisen, die parallel zueinander sind. Ferner kann die Randfläche zwei gegenüberliegende Stirnseiten aufweisen, die jeweils in Draufsicht gesehen abgerundet ausgebildet sind. Ferner können die Scheitellinien der abgerundeten Stirnseiten um 90°zueinander verkippt sein.

Die Ausbildung einer solchen Randfläche führt zu einer leichteren Herstellbarkeit und einer leichteren Prüfbarkeit des hergestellten Deckglases.

Bei dem erfindungsgemäßen Endoskop kann die Abbildungsoptik mehrere Linsen aufweisen, wobei die am nähesten am Deckglas positionierte Linse der Abbildungsoptik als Plan-Konkav-Linse, deren plane Seite dem Deckglas zugewandt ist, oder als Konkav-Konkav-Linse ausgebildet ist, wobei dann die dem Deckglas zugewandte Seite natürlich konkav gekrümmt ist.

Da die plane oder die konkave Seite dem Deckglas zugewandt ist, ist in vorteilhafter Weise nahe am Deckglas eine Negativ-Linse mit hoher Brechkraft positioniert, wodurch die radiale Ausdehnung der Abbildungsoptik bei gleichbleibenden bzw. nahezu gleichbleibenden Abbildungseigenschaften verringert werden kann.

Das erfindungsgemäße Endoskop kann insbesondere als starres Endoskop (also als Endoskop mit einem starren Endoskopschaft) ausgebildet sein. Es ist jedoch auch möglich, daß der Endoskopschaft zumindest in einem Abschnitt abwinkelbar ist.

Ferner kann das Endoskop am Handgriff ein Okular aufweisen und/oder eine Schnittstelle für z.B. eine Videokamera. Alternativ ist es möglich, daß das Endoskop am distalen Ende einen der Abbildungsoptik nachgeordneten Bildsensor enthält.

Das Endoskop kann weitere, dem Fachmann bekannte Elemente aufweisen, die zum Betrieb des Endoskops notwendig sind.

Es versteht sich, daß die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Nachfolgend wird die Erfindung beispielsweise anhand der beigefügten Zeichnungen, die auch erfindungswesentliche Merkmale offenbaren, noch näher erläutert. Es zeigen:
- Fig. 1: eine perspektivische Ansicht einer Ausführungsform des erfindungsgemäßen Endoskops;
- Fig. 2: eine vergrößerte Schnittansicht des distalen Endes des Endoskopschaftes 3;
- Fig. 3: eine erste perspektivische Ansicht des Deckglases 7;
- Fig. 4: eine zweite perspektivische Ansicht des Deckglases 7;
- Fig. 5: eine Seitenansicht des Deckglases 7;
- Fig. 6: eine Ansicht des Deckglases entlang der Richtung des Pfeils P1 in Figur 5;
- Fig. 7: eine Ansicht des Deckglases entlang der Richtung des Pfeils P2 in Figur 5;
- Fig.8: eine vergrößerte Schnittdarstellung des Deckglases entlang der Schnittlinie A-A aus Figur 5;
- Fig. 9: eine vergrößerte Schnittdarstellung des Deckglases entlang der Schnittlinie B-B aus Figur 7, und
- Fig. 10: eine schematische Schnittansicht von Deckglas und Abbildungsoptik eines erfindungsgemäßen Endoskops gemäß einer weiteren Ausführungsform.

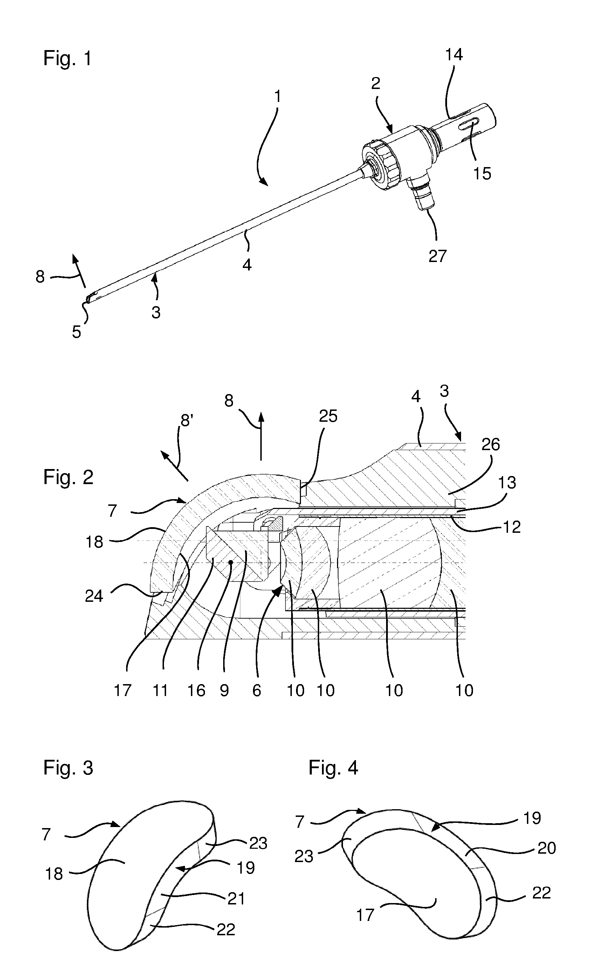

Bei der in Fig. 1 gezeigten Ausführungsform ist das erfindungsgemäße Endoskop 1 als Endoskop 1 mit variabler Blickrichtung ausgebildet und weist einen Handgriff 2 sowie einen mit dem Handgriff 2 verbundenen Endoskopschaft 3 auf, dessen Mantelrohr 4 in Fig. 1 sichtbar ist.

Wie insbesondere der vergrößerten Schnittdarstellung des distalen Endes 5 des Endoskopschaftes 3 in Figur 2 zu entnehmen ist, ist im Endoskopschaft 3 eine Abbildungsoptik 6 angeordnet und ist am distalen Ende 5 ein Deckglas 7 positioniert. Mit der Abbildungsoptik 6 kann ein in Blickrichtung 8 der Abbildungsoptik 6 vor dem Deckglas 7 befindliches Objekt als Bild abgebildet werden.

Die Abbildungsoptik 6 umfaßt ein Umlenkprisma 9 sowie diesem nachgeordnete Linsen 10. Das Umlenkprisma 9 sitzt in einem Prismenhalter 11, der schwenkbar am distalen Ende eines im Endoskopschaft 3 angeordneten Optikrohrs 12 gelagert ist.

Das Optikrohr 12 ist in einem Zugrohr 13 positioniert und das Zugrohr 13 ist in Längsrichtung des Endoskopschafts 3 relativ zum Optikrohr 12 und zum Mantelrohr 4 verschiebbar gelagert, wobei die axiale Stellung des Zugrohrs 13 mittels eines am Handgriff 2 angeordneten Betätigungselementes 14 einstellbar ist. Das Betätigungselement 14 ist hier als Hülse ausgebildet, die um die Längsachse des Endoskopschafts 3 drehbar gelagert, und weist Vertiefungen 15 zu besseren Handhabbarkeit auf. Eine Drehung des Betätigungselementes 14 über eine nicht gezeigte Koppelmechanik führt zu einer axialen Verschiebung des Zugrohrs 13. Das distale Ende des Zugrohrs 13 ist so mit dem Prismenhalter 11 gekoppelt, daß eine axiale Verschiebung des Zugrohrs zu einem Drehen des Prismenhalters 11 und somit des Umlenkprismas 9 um die in Figur 2 schematisch angezeigte Drehachse 16 führt. Diese Drehung des Umlenkprismas 9 führt dazu, daß die Blickrichtung 8 verändert wird, beispielsweise zu der schematisch in Figur 2 eingezeichneten Blickrichtung 8'.

Die Abbildungsoptik 6 weist hier noch ein Bildübertragungssystem in der Form von nicht gezeigten Stablinsen im Endoskopschaft 3 auf, das dazu dient, das aufgenommene Bild bis zum proximalen Ende des Handgriffs 2 zu übertragen, wo es dann bereitgestellt ist. Das bereitgestellte Bild kann direkt oder über ein proximal angeordnetes Okular (beispielsweise innerhalb des Handgriffs 2) betrachtet werden. Auch ist es möglich, eine Videokamera am proximalen Ende des Handgriffs 2 anzubringen, die das Bild aufnimmt und über eine Ausgabeeinheit (beispielsweise einen Monitor) darstellen kann.

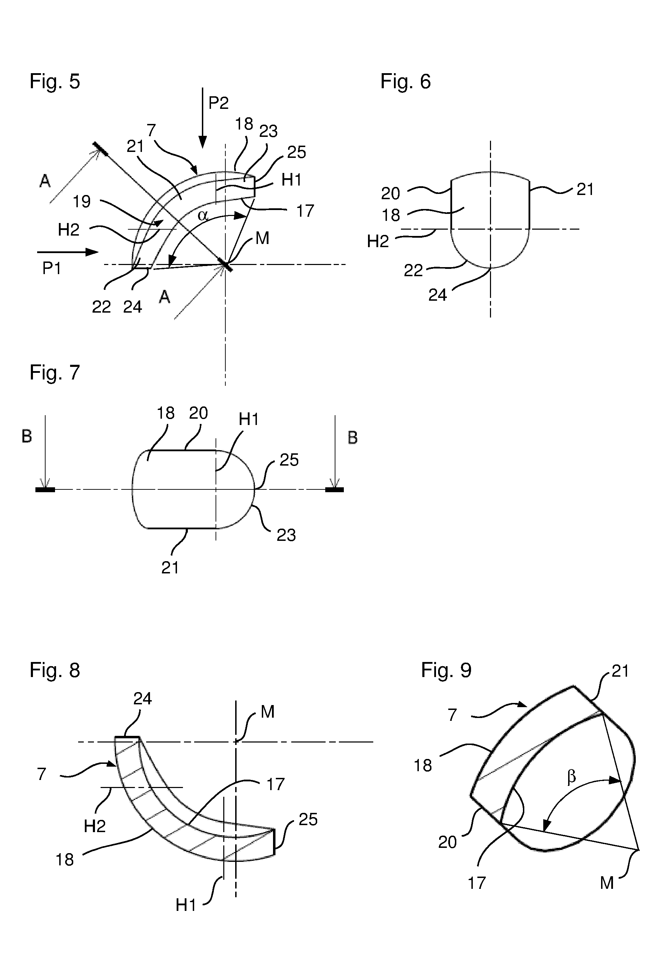

Das Deckglas 7 weist eine sphärisch gekrümmte, konkave Innenseite 17 und eine sphärisch gekrümmte, konvexe Außenseite 18 auf, wobei die Krümmungsmittelpunkte von Innen- und Außenseite 17, 18 zusammenfallen (in Figuren 5, 8 und 9 als Punkt M eingezeichnet), so daß die Dicke des Deckglases 7 konstant ist. Unter Dicke wird hier insbesondere der Abstand von Innen- und Außenseite in radialer Richtung (also ausgehend von den Krümmungsmittelpunkten) verstanden. Das Deckglas 7 kann daher auch als Kuppelglas bezeichnet werden.

Diese Ausbildung des Deckglases 7 führt zu dem großen Vorteil, daß die von jedem Objektpunkt ausgehenden und für die Bilderzeugung benutzten Strahlen senkrecht bzw. im wesentlichen senkrecht durch das Deckglas 7 hindurch treten. Daher kann das Deckglas 7 als für die Abbildung im wesentlichen neutrales Element (das im wesentlichen keine optischen Abbildungseigenschaften aufweist) angesehen werden, so daß beim Durchtritt der Strahlen durch das Deckglas 7 keine bzw. im wesentlichen vernachlässigbare Brechung auftritt, und zwar unabhängig vom an der Außenseite 18 des Deckglases 7 vorhandenen Medium sowie unabhängig von der durch die Schwenkstellung des Umlenkprismas 9 eingestellten Blickrichtung 8, 8'. Besonders gut kann dieser Vorteil genutzt werden, wenn die Krümmungsmittelpunkte der Innen- und Außenseite 17, 18 auf oder nahe der Schwenkachse 16 liegen, wobei die Schwenkachse 16 bevorzugt in der Seite des Umlenkprismas 9 liegt, die die Strahlumlenkung bewirkt. Natürlich kann von diesen für die optische Abbildung optimalen Bedingungen auch mehr oder minder stark abgewichen werden, wenn dies z.B. aufgrund des vorhandenen Raumes für die Abbildungsoptik 6 und insbesondere für die Umlenkung des Umlenkprismas 9 notwendig ist.

Aufgrund des für die Abbildung benutzten Öffnungswinkels der von jedem Objektpunkt ausgehenden Strahlen kann zwar eine Brechung nicht für alle Strahlen verhindert werden. Sie ist jedoch sehr viel geringer im Vergleich zu anderen Formen des Deckglases, wie z.B. bei einer Ausbildung des Deckglases als planparallele Platte. Würde als Deckglas eine planparallele Platte verwendet werden, wäre eine sehr starke Abhängigkeit der Brechung einerseits vom umgebenen Medium vorhanden (z.B. Luft oder eine Flüssigkeit) und andererseits auch eine starke Abhängigkeit von der gerade gewählten Blickrichtung 8, 8'.

In Figuren 3 und 4 sind perspektivische Ansichten des Deckglases 7 dargestellt. Aus diesen Darstellungen ist ersichtlich, daß das Deckglas 7 in Draufsicht gesehen nicht mehr rund, wie bisher üblich ist, sondern eher eine längliche Form mit abgerundeten Schmalseiten aufweist.

In Figur 5 ist das Deckglas in Seitenansicht dargestellt, in Figur 6 ist die Ansicht entlang der Richtung des Pfeils P1 und in Figur 7 ist die Ansicht entlang der Richtung des Pfeils P2 gezeigt. Figur 8 zeigt die vergrößerte Schnittdarstellung A-A gemäß Figur 5 und Figur 9 zeigt die vergrößerte Schnittdarstellung B-B gemäß Figur 7.

Wie der Darstellung in Figur 5 zu entnehmen ist, erstreckt sich das Deckglas 7 über den Winkelbereich α in einer ersten Ebene und erstreckt sich das Deckglas 7 in einer dazu senkrechten, zweiten Ebene über den Winkelbereich β (Figur 9), wobei der erste Winkelbereich α größer ist als der zweite Winkelbereich β. Aufgrund dieser unterschiedlichen Winkelbereiche α, β ergibt sich die beschriebene längliche Form des Deckglases 7. Bei der hier beschriebenen Ausführungsform ist der erste Winkelbereich α fast doppelt so groß wie der zweite Winkelbereich β.

Die Innen- und Außenseite 17 und 18 sind mit einem Rand 19, der als ebene Randfläche ausgebildet ist, verbunden. Die Ausrichtung der Randfläche 19 ist jedoch nicht radial ausgehend von den Krümmungsmittelpunkten der Innen- und Außenseite 17, 18, sondern zu dieser radialen Richtung geneigt.

So weist die Randfläche 19 einen linken und rechten Abschnitt 20, 21 an den Längsseiten des Deckglases 7 auf, die sich zueinander parallel strecken. Der linke und rechte Abschnitt 20, 21 sind über einen unteren Stirnabschnitt 22 und einen oberen Stirnabschnitt 23 miteinander verbunden, wobei im Bereich der beiden Stirnabschnitte 22 und 23 die Außenseite 18 in Draufsicht gesehen abgerundet ist, wie insbesondere in Figuren 6 und 7 ersichtlich ist. Gleiches gilt natürlich, in einer Ansicht von unten gesehen, für die Innenseite 17. Die beiden Stirnabschnitte 22 und 23 sind je so ausgebildet, daß ihre Scheitellinien (untere Scheitellinie 24 beim unteren Stirnabschnitt 22 und obere Scheitellinie 25 beim oberen Stirnabschnitt 23) um 90° zueinander verkippt sind. Die Randfläche 19 kann somit auch als gerade Randfläche bezeichnet werden. Diese Ausbildung der Randfläche 19 erleichtert die Herstellung des Deckglases 7 sowie auch die noch nachfolgend beschriebene Verbindung des Deckglases 7 mit einem Innenrohr 26 des Endoskops 1. Die Übergangsstelle zwischen dem linken und rechten Abschnitt 20, 21 mit dem jeweiligen Stirnabschnitt 22 und 23 ist in Figuren 5-8 durch Hilfslinien H1 und H2 dargestellt.

Die Innenseite 17 des Deckglases 7, das hier als Saphir-Deckglas ausgebildet ist, ist mit einer Entspiegelungsbeschichtung versehen und somit entspiegelt. Natürlich kann zusätzlich oder alternativ die Außenseite 18 entspiegelt sein.

Das Deckglas 7 ist hier mit seinem Rand 19 mit dem distalen Ende des Innenrohrs 26 so verlötet, daß die Öffnung am distalen Ende des Innenrohrs 26 hermetisch dicht verschlossen ist (Fig. 2). Innerhalb des Innenrohrs 26 sitzt das Zugrohr 13 und das Innenrohr 26 ist seinerseits vom Mantelrohr 4 umgeben, wobei in üblicher Art und Weise zwischen dem Innenrohr 26 und dem Mantelrohr 4 zumindest ein sich in axialer Richtung des Mantelrohrs 4 erstreckender Zwischenraum vorhanden ist, in dem (nicht gezeigte) Lichtleitfasern angeordnet sind, die zur Beleuchtung des abzubildenden Objektes dienen. Die Lichtleitfasern können über einen Lichtleiteranschluß 27 am Handgriff 2 mit Licht beaufschlagt werden.

Um das Verlöten des Deckglases 7 mit dem Innenrohr 26 zu erleichtern, kann der Rand 19 mit einer lötfähigen Metallschicht versehen sein.

Natürlich ist es auch möglich, daß das Deckglas 7 mit dem distalen Ende des Innenrohrs 26 verklebt ist oder in sonstiger Art und Weise verbunden ist.

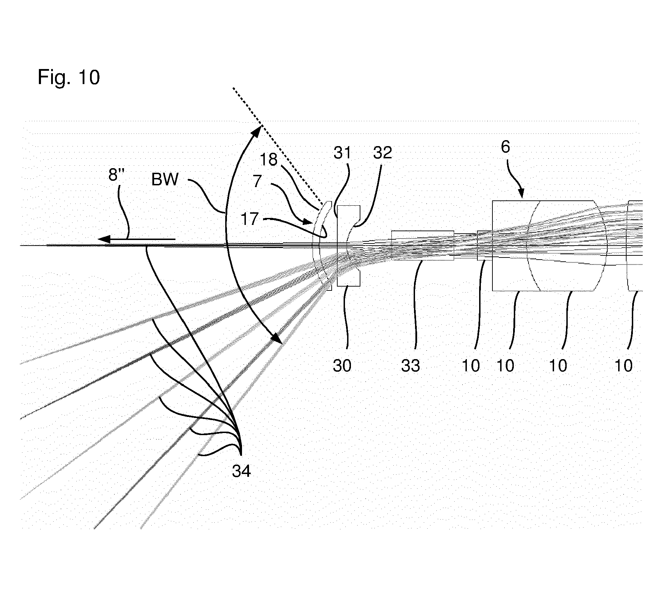

In Figur 10 ist in schematischer Schnittansicht das Deckglas 7 sowie die Abbildungsoptik 6 einer weiteren Ausführungsform des erfindungsgemäßen Endoskopes 1 gezeigt.

Im Unterschied zu der in Verbindung mit Figuren 1 bis 9 beschriebenen Ausführungsform weist die Abbildungsoptik 6 bei der Variante gemäß Figur 10 eine Plan-Konvex-Linse 30 auf, die als Negativ-Linse ausgebildet und von allen Linsen 30, 10 der Abbildungsoptik 6 am nähesten zum Deckglas 7 positioniert ist, wobei die plane Seite 31 der Linse 30 dem Deckglas 7 zugewandt und die konkave Seite 32 der Linse 30 dem Deckglas 7 abgewandt ist.

Ferner sind in Figur 10 noch beispielhaft einige Lichtbündel 34 dargestellt, die den halben Bildwinkel BW andeuten, der mit dem Endoskop 1 aufnehmbar ist.

Bei der Ausführungsform gemäß Figur 10 sind die Innen- und Außenseite 17, 18 wiederum sphärisch gekrümmt, wobei ihre Krümmungsmittelpunkte zusammenfallen. Insbesondere ist das Endoskop so ausgebildet, daß die beiden Krümmungsmittelpunkte mit der Eintrittspupille der Abbildungsoptik 6 zusammenfallen. Die Lage der Eintrittspupille wird durch den Schnittpunkt aller Hauptstrahlen (bzw. mittlerer Lichtstrahl der dargestellten Lichtbündel 34) bestimmt und ist idealerweise ein Punkt. Durch das Vorhandensein von Pupillenaberrationen in der realen Abbildungsoptik 6 ist die Eintrittspupille meist leicht gekrümmt und nicht genau in einem Punkt zu finden. In diesem Fall wird bezüglich der Lage der Krümmungsmittelpunkte von Innen- und Außenseite 17, 18 des Deckglases 7 ein Kompromiß gesucht. Z. B. können die Krümmungsmittelpunkte in der Mitte der verschiedenen Schnittpunkte der Hauptstrahlen liegen. Die Krümmung der Außenseite 18 des Deckglases 7 wird insbesondere so gewählt, daß die Hauptstrahlen aller Lichtbündel aus dem Bildwinkel BW rechtwinklig auf die Außenseite 18 treffen.

Die Wandstärke des Deckglases 7 kann z. B. im Bereich von 0,1 bis 0,3 mm liegen. Bei der hier beschriebenen Ausführungsform beträgt die Wandstärke beispielsweise 0,2 mm.

Mit einem solchen Deckglas, das auch als Nullinse bezeichnet werden kann, ist es möglich, daß der Bildwinkel des Endoskopes sich nicht oder nur kaum ändert, wenn das Deckglas in Luft oder z. B. in einer Kochsalzlösung oder einem sonstigen anderen transparenten Medium, insbesondere einem transparenten flüssigen Medium ist. Aufgrund der planen Seite 31 weist die distale Linse 30 eine höhere Brechung auf im Vergleich zu einer distalen Linse, bei der die dem Deckglas 7 zugewandte Seite konvex gekrümmt ausgebildet ist, wodurch die Abbildungsoptik 6 insgesamt mit geringerem Durchmesser bei gleichem Bildwinkel BW ausgebildet werden kann.

Das Deckglas 7 ist insbesondere aus Glas oder Kristall gebildet. Beispielsweise kann das Deckglas als Saphir-Deckglas ausgebildet sein. Insbesondere ist das Deckglas aus einem chemisch inerten Material gebildet, das z. B. eine vorbestimmte Härte aufweist.

Bei der in Figur 10 gezeigten Darstellung wurde zunächst davon ausgegangen, daß eine Blickrichtung 8" von 0° vorliegt. Die Blickrichtung 8" verläuft in diesem Fall parallel zur Längsachse des Endoskopschaftes 3 (Fig. 1) oder parallel zur optischen Achse der Abbildungsoptik 6. Für Endoskope, die eine von 0° verschiedene Blickrichtungen aufweisen, kann der optische Aufbau gemäß Figur 10 in gleicher Weise eingesetzt werden. In diesem Fall ist das mit dem Bezugszeichen 33 schematisch eingezeichnete Umlenkelement (beispielsweise Prisma) vorgesehen.

In einer Abwandlung kann die distale Linse als Konkav-Konkav-Linse ausgebildet sein, wobei eine solche Ausbildung wiederum vorteilhaft zu einer distalen Linse führt, deren dem Deckglas zugewandte Seite konkav gekrümmt ist.

Natürlich kann auch bei der in Verbindung mit Figuren 1 bis 9 beschriebenen Ausführungsform die distale Linse 10 der Abbildungsoptik 6 (also die Linse 10, die am nähesten zum Deckglas 7 positioniert ist) als Plan-Konkav-Linse 30, wobei die plane Seite 31 dem Deckglas 7 zugewandt ist, oder als Konkav-Konkav-Linse 30, wobei eine der konkaven Seiten dem Deckglas 7 zugewandt ist, ausgebildet sein. Die distale Linse 30 kann in diesem Fall entweder zwischen dem Umlenkprisma 9 und dem Deckglas 7 positioniert sein oder kann dem Umlenkprisma 9 nachgeordnet sein, so daß in diesem Fall die Lichtstrahlen erst durch das Deckglas 7 laufen, mittels dem Umlenkprisma 9 umgelenkt werden und dann auf die distale Linse 30 treffen.

## Patentansprüche

1. Endoskop mit
einem ersten Rohr (20),
einem am distalen Ende des ersten Rohrs (20) angeordneten Deckglas (7) sowie mit einer im ersten Rohr (20) angeordneten Abbildungsoptik (6), die ein vor dem Deckglas (7) befindliches Objekt als Bild abbildet,
wobei das Deckglas (7) eine gekrümmte, konkave Innenseite (17), eine gekrümmte, konvexe Außenseite (18) sowie eine konstante Dicke aufweist,
**dadurch gekennzeichnet, daß**
die Innen- und Außenseite (17, 18) jeweils sphärisch gekrümmt ist und
daß das Deckglas (7) eine die Innen- und Außenseite (17, 18) verbindende, ebene Randfläche (19) aufweist, die gegenüber dem Krümmungsradius der Innen- und Außenseite (17, 18) geneigt ist.

2. Endoskop nach Anspruch 1, **dadurch gekennzeichnet, daß** sich das Deckglas in einer ersten Ebene über einen ersten Winkelbereich (α) erstreckt und sich in einer zweiten Ebene, die senkrecht zur ersten Ebene ist, über einen zweiten Winkelbereich (β) erstreckt, wobei der erste Winkelbereich (α) größer ist als der zweite Winkelbereich (β).

3. Endoskop nach Anspruch 2, **dadurch gekennzeichnet, daß** der erste Winkelbereich (α) mindestens 1,5 mal so groß ist wie der zweite Winkelbereich (β).

4. Endoskop nach einem der obigen Ansprüche, **dadurch gekennzeichnet, daß** die Innenseite (17) und/oder die Außenseite (18) entspiegelt sind/ist.

5. Endoskop nach einem der obigen Ansprüche, **dadurch gekennzeichnet, daß** das Deckglas als Saphir-Deckglas ausgebildet ist.

6. Endoskop nach einem der obigen Ansprüche, **dadurch gekennzeichnet, daß** der Rand (19) des Deckglases (7) mit dem distalen Ende des ersten Rohrs (20) verlötet ist.

7. Endoskop nach einem der obigen Ansprüche, **dadurch gekennzeichnet, daß** die Abbildungsoptik (6) ein schwenkbar gelagertes Umlenkelement (9) aufweist, mit dem die Blickrichtung durch das Deckglas (7) einstellbar ist.

8. Endoskop nach Anspruch 7, **dadurch gekennzeichnet, daß** die Mittelpunkte der Krümmungsradien der Innen- und Außenseite (17, 18) zusammenfallen und auf der Schwenkachse des Umlenkelementes (9) liegen.

9. Endoskop nach Anspruch 7 oder 8, **dadurch gekennzeichnet, daß** das Umlenkelement (9) eine eine Strahlumlenkung bewirkende Fläche aufweist, die Mittelpunkte der Krümmungsradien der Innen- und Außenseite (17, 18) zusammenfallen und in der die Strahlumlenkung bewirkende Fläche liegen.

10. Endoskop nach einem der obigen Ansprüche, **dadurch gekennzeichnet, daß** die Randfläche (19) einen linken und rechten Abschnitt (20, 21) aufweist, die parallel zueinander sind.

11. Endoskop nach einem der obigen Ansprüche, **dadurch gekennzeichnet, daß** die Randfläche (19) zwei gegenüberliegende Stirnabschnitte (22, 23) aufweist, die jeweils in Draufsicht gesehen abgerundet sind.

12. Endoskop nach Anspruch 11, **dadurch gekennzeichnet, daß** die Scheitellinien (24, 25) der abgerundeten Stirnabschnitte (22, 23) um 90° zueinander verkippt sind.

13. Endoskop nach einem der obigen Ansprüche, **dadurch gekennzeichnet, daß** die Abbildungsoptik (6) mehrere Linsen (10, 30) aufweist, wobei die am nähesten am Deckglas (7) positionierte Linse (30) der Abbildungsoptik (6) als Plan-Konkav-Linse (30) ausgebildet ist, deren plane Seite (31) dem Deckglas (7) zugewandt ist.

14. Endoskop nach einem der Ansprüche 1 bis 12, **dadurch gekennzeichnet, daß** die Abbildungsoptik (6) mehrere Linsen (10, 30) aufweist, wobei die am nähesten am Deckglas (7) positionierte Linse (30) der Abbildungsoptik (6) als Konkav-Konkav-Linse (30) ausgebildet ist.

## Claims

1. An endoscope, comprising
a first tube (20), and
a cover glass (7) arranged at the distal end of the first tube (20), as well as with imaging optics (6), arranged in the first tube (20), which image an object located in front of the cover glass (7) as an image,
wherein the cover glass (7) has a curved, concave inside (17), a curved, convex outside (18), and a constant thickness,
**characterized in that**
the inside (17) and the outside (18) are each spherically curved, and
the cover glass (7) comprises a flat lateral face (19), connecting the inside (17) and the outside (18), which is inclined relative to the radius of curvature of the inside (17) and the outside (18).

2. The endoscope according to claim 1, wherein the cover glass extends, in a first plane, over a first angle range (α) and, in a second plane which is perpendicular to the first plane, over a second angle range (β), wherein the first angle range (α) is larger than the second angle range (β).

3. The endoscope according to claim 2, wherein the first angle range (α) is at least 1.5 times as large as the second angle range (β).

4. The endoscope according to one of the above claims, wherein at least one of the inside (17) and the outside (18) are antireflection-coated.

5. The endoscope according to one of the above claims, wherein the cover glass is formed as a sapphire cover glass.

6. The endoscope according to one of the above claims, wherein the edge (19) of the cover glass (7) is soldered to the distal end of the first tube (20).

7. The endoscope according to one of the above claims, wherein the imaging optics (6) include a swivellably housed deflecting element (9) with which the viewing direction through the cover glass (7) can be set.

8. The endoscope according to claim 7, wherein the centres of the radii of curvature of the inside (17) and the outside (18) coincide and lie on the swivel axis of the deflecting element (9).

9. The endoscope according to claim 7 or 8, wherein the deflecting element (9) includes a surface that brings about a beam deflection, the centres of the radii of curvature of the inside (17) and the outside (18) coincide and lie in the surface that brings about the beam deflection.

10. The endoscope according to one of the above claims, wherein the lateral face (19) has a left and a right section (20, 21) which are parallel to each other.

11. The endoscope according to one of the above claims, wherein the lateral face (19) has two opposite end sections (22, 23) which, in each case seen in top view, are rounded.

12. The endoscope according to claim 11, wherein the vertex lines (24, 25) of the rounded end sections (22, 23) are tilted towards each other by 90°.

13. The endoscope according to one of the above claims, wherein the imaging optics (6) include several lenses (10, 30), wherein the lens (30) of the imaging optics (6) positioned closest to the cover glass (7) is formed as a flat-concave lens the flat side (31) of which faces the cover glass (7).

14. The endoscope according to claims 1 to 12, wherein the imaging optics (6) include several lenses (10, 30), wherein the lens (30) of the imaging optics (6) positioned closest to the cover glass (7) is formed as a concave-concave lens (30).

## Revendications

1. Endoscope comprenant
un premier tube (20),
une calotte en verre (7) disposée à l'extrémité distale du premier tube (20) ainsi
qu'une optique de représentation (6) disposée dans le premier tube (20), laquelle représente en tant qu'image un objet qui se trouve devant la calotte en verre (7), la calotte en verre (7) possédant un côté intérieur (17) concave courbe, un côté extérieur (18) convexe courbe ainsi qu'une épaisseur constante,
**caractérisé en ce que**
les côtés extérieur et intérieur (17, 18) possèdent respectivement une courbure sphérique et
**en ce que** la calotte en verre (7) possède une surface de bordure (19) plane, reliant les côtés extérieur et intérieur (17, 18), laquelle est inclinée par rapport au rayon de courbure des côtés extérieur et intérieur (17, 18).

2. Endoscope selon la revendication 1, **caractérisé en ce que** la calotte en verre s'étend dans un premier plan sur une première plage angulaire (α) et s'étend dans un deuxième plan, lequel est perpendiculaire au premier plan, sur une deuxième plage angulaire (ß), la première plage angulaire (α) étant plus grande que la deuxième plage angulaire (ß).

3. Endoscope selon la revendication 2, **caractérisé en ce que** la première plage angulaire (α) est au moins 1,5 fois plus grande que la deuxième plage angulaire (ß).

4. Endoscope selon l'une des revendications précédentes, **caractérisé en ce que** le côté intérieur (17) et/ou le côté extérieur (18) sont/est antireflet.

5. Endoscope selon l'une des revendications précédentes, **caractérisé en ce que** la calotte en verre est réalisée sous la forme d'une calotte en verre en saphir.

6. Endoscope selon l'une des revendications précédentes, **caractérisé en ce que** le bord (19) de la calotte en verre (7) est brasé avec l'extrémité distale du premier tube (20).

7. Endoscope selon l'une des revendications précédentes, **caractérisé en ce que** l'optique de représentation (6) possède un élément de renvoi (9) monté pivotant à l'aide duquel peut être réglé le sens d'observation à travers la calotte en verre (7).

8. Endoscope selon la revendication 7, **caractérisé en ce que** les points centraux des rayons de courbure des côtés extérieur et intérieur (17, 18) coïncident et se trouvent sur l'axe de pivotement de l'élément de renvoi (9).

9. Endoscope selon la revendication 7 ou 8, **caractérisé en ce que** l'élément de renvoi (9) possède une surface provoquant un renvoi du rayon, les points centraux des rayons de courbure des côtés extérieur et intérieur (17, 18) coïncident et se trouvent dans la surface provoquant le renvoi du rayon.

10. Endoscope selon l'une des revendications précédentes, **caractérisé en ce que** la surface de bordure (19) possède une portion gauche et droite (20, 21) qui sont parallèles l'une à l'autre.

11. Endoscope selon l'une des revendications précédentes, **caractérisé en ce que** la surface de bordure (19) possède deux portions frontales (22, 23) opposées qui, vues de dessus, sont respectivement arrondies.

12. Endoscope selon la revendication 11, **caractérisé en ce que** les lignes de sommet (24, 25) des portions frontales (22, 23) arrondies présentent un désalignement angulaire de 90° entre elles.

13. Endoscope selon l'une des revendications précédentes, **caractérisé en ce que** l'optique de représentation (6) possède plusieurs lentilles (10, 30), la lentille (30) de l'optique de représentation (6) qui est positionnée le plus près de la calotte en verre (7) étant réalisée sous la forme d'une lentille plan-concave (30) dont le côté plan (31) fait face à la calotte en verre (7).

14. Endoscope selon l'une des revendications 1 à 12, **caractérisé en ce que** l'optique de représentation (6) possède plusieurs lentilles (10, 30), la lentille (30) de l'optique de représentation (6) qui est positionnée le plus près de la calotte en verre (7) étant réalisée sous la forme d'une lentille concave-concave (30).
